# EUROPÄISCHE PATENTSCHRIFT

(11) **EP 0 725 822 B1**
(45) Veröffentlichungstag und Bekanntmachung des Hinweises auf die Patenterteilung: **10.05.2000**
(21) Anmeldenummer: 94929554.7
(22) Anmeldetag: 17.10.1994
(51) Int. Cl.: C12N 15/16, C12N 15/81

(54) **VERFAHREN ZUR REKOMBINANTEN HERSTELLUNG VON PROTEINEN IN HEFE**
PROCESS FOR THE RECOMBINANT PRODUCTION OF PROTEINS IN YEAST
PROCEDE POUR LA PRODUCTION DE PROTEINES PAR RECOMBINAISON DANS UNE LEVURE

(30) Priorität: 28.10.1993 DE 4336810
(43) Veröffentlichungstag der Anmeldung: 14.08.1996
(73) Patentinhaber: BASF AKTIENGESELLSCHAFT, 67056 Ludwigshafen (DE); RHEIN BIOTECH GESELLSCHAFT FUR NEUE BIOTECHNOLOGISCHE PROZESSE UND PRODUKTE, 40595 Düsseldorf (DE)
(72) Erfinder: SCHWEDEN, Jürgen, D-67433 Neustadt (DE); BOLLSCHWEILER, Claus, D-69118 Heidelberg (DE); PIONTEK, Michael, D-45136 Essen (DE); WEYDEMANN, Ulrike, D-50825 Köln (DE); GELLISSEN, Gerd, D-42489 Wülfrath (DE)
(86) Internationale Anmeldenummer: EP9403409
(87) Internationale Veröffentlichungsnummer: WO9511976

(56) Entgegenhaltungen:
- EP-A- 0 121 884
- WO-A-88/07086
- ANTONIE VAN LOEWENHOEK, Bd.62, 1992 Seiten 79 - 93 GELLISEN G. ET AL. 'Heterologous protein production in yeast'
- FEBS LETTERS, Bd.257, Nr.1, 1989 Seiten 31 - 34 WEIDEMANN W. ET AL. 'Cloning and sequence analysis of cDNA for precursor of a crustacean hyperglycemic hormone'
- P.N.A.S.,, Bd.84, 1987 Seiten 3043 - 3046 HIROSHI NOJIRI ET AL. 'Cloning and sequence ananlysis of cDNAS for neurohypophysial hormones vasotocin and mesotocin for the hypothalamus of toad'

SEQUENZPROTOKOLL

(1) ALLGEMEINE INFORMATION:

(i) ANMELDER:
(A) NAME: BASF Aktiengesellschaft
(B) STRASSE: Carl-Bosch-Strasse 38
(C) ORT: Ludwigshafen
(E) LAND: Bundesrepublik Deutschland
(F) POSTLEITZAHL: D-67056
(G) TELEPHON: 0621/6048526
(H) TELEFAX: 0621/6043123
(I) TELEX: 1762175170
(A) NAME: Rhein Biotech GmbH
(B) STRASSE: Eichsfelder Strasse 11
(C) ORT: Duesseldorf
(E) LAND: Bundesrepublik Deutschland
(F) POSTLEITZAHL: D-40595
(G) TELEPHON: 0211/709010
(H) TELEFAX: 0211/7090130

(ii) ANMELDETITEL: Verfahren zur rekombinanten Herstellung von
Proteinen in Hefe

(iii) ANZAHL DER SEQUENZEN: 8

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 142 Aminosäuren
(B) ART: Aminosäure
(D) TOPOLOGIE: linear

(ii) ART DES MOLEKÜLS: Peptid

(iii) HYPOTHETISCH: NEIN

(iii) ANTISENSE: NEIN
(v) ART DES FRAGMENTS: N-Terminus

(vi) URSPRÜNLICHE HERKUNFT:
(A) ORGANISMUS: Carcinus maenas

(ix) MERKMALE:
(A) NAME/SCHLÜSSEL: Peptide
(B) LAGE: 1..26
(D) SONSTIGE ANGABEN: /label= Leader

(ix) MERKMALE:
(A) NAME/SCHLÜSSEL: Peptide
(B) LAGE: 27..66
(D) SONSTIGE ANGABEN: /label= Adaptor

(ix) MERKMALE:
(A) NAME/SCHLÜSSEL: Peptide
(B) LAGE: 67..142
(D) SONSTIGE ANGABEN: /label= CHH

(xi) SEQUENZBESCHREIBUNG: SEQ ID NO: 1:

Met Tyr Ser Lys Thr Ile Pro Ala Met Leu Ala Ile Ile Thr Val Ala
1               5                   10                  15
Tyr Leu Cys Ala Leu Pro His Ala His Ala Arg Ser Thr Gln Gly Tyr
            20                  25                  30
Gly Arg Met Asp Arg Ile Leu Ala Ala Leu Lys Thr Ser Pro Met Glu
        35                  40                  45
Pro Ser Ala Ala Leu Ala Val Glu Asn Gly Thr Thr His Pro Leu Glu
    50                  55                  60
Lys Arg Gln Ile Tyr Asp Thr Ser Cys Lys Gly Val Tyr Asp Arg Ala
65                  70                  75                  80
Leu Phe Asn Asp Leu Glu His Val Cys Asp Asp Cys Tyr Asn Leu Tyr
                85                  90                  95
Arg Thr Ser Tyr Val Ala Ser Ala Cys Arg Ser Asn Cys Tyr Ser Asn
            100                 105                 110
Leu Val Phe Arg Gln Cys Met Asp Asp Leu Leu Met Met Asp Glu Phe
        115                 120                 125
Asp Gln Tyr Ala Arg Lys Val Gln Met Val Gly Arg Lys Lys
    130                 135                 140

(2) INFORMATION ZU SEQ ID NO: 2:

(i) SEQUENZ CHARAKTERISTIKA:
(A) LÄNGE: 65 Aminosäuren
(B) ART: Aminosäure
(C) STRANGFORM: Einzel
(D) TOPOLOGIE: linear

(ii) ART DES MOLEKÜLS: Protein

(iii) HYPOTHETISCH: NEIN

(iii) ANTISENSE: NEIN

(xi) SEQUENZBESCHREIBUNG: SEQ ID NO: 2:

Val Val Tyr Thr Asp Cys Thr Glu Ser Gly Gln Asn Leu Cys Leu Cys
1               5                   10                  15
Glu Gly Ser Asn Val Cys Gly Gln Gly Asn Lys Cys Ile Leu Gly Ser
            20                  25                  30
Lys Gly Glu Arg Asn Gln Cys Val Thr Gly Glu Gly Thr Pro Arg Pro
        35                  40                  45
Gln Ser His Asn Asp Gly Asp Phe Glu Glu Ile Pro Glu Glu Tyr Leu
    50                  55                  60
Gln
65

(2) INFORMATION ZU SEQ ID NO: 3:

(i) SEQUENZ CHARAKTERISTIKA:
(A) LÄNGE: 140 Basenpaare
(B) ART: Nukleinsäure
(C) STRANGFORM: Einzel
(D) TOPOLOGIE: linear

(ii) ART DES MOLEKÜLS: DNS (genomisch)

(xi) SEQUENZBESCHREIBUNG: SEQ ID NO: 3:

GGGGGGGAAT TCATGTATAG CAAAACTATT CCCGCCATGC TAGCAATCAT CACCGTAGCC 60
TACCTATGCG CACTCCCGCA CGCACACGCA CGCTCCACGC AAGGCTACGG ACGCATGGAT 120
CGCATCCTGG CGGCCTTGAA 140

(2) INFORMATION ZU SEQ ID NO: 4:

(i) SEQUENZ CHARAKTERISTIKA:
(A) LÄNGE: 122 Basenpaare
(B) ART: Nukleinsäure
(C) STRANGFORM: Einzel
(D) TOPOLOGIE: linear

(ii) ART DES MOLEKÜLS: DNS (genomisch)

(xi) SEQUENZBESCHREIBUNG: SEQ ID NO: 4:

GGGGGGGATT CAGTGCAGTC AGTGTAAACA ACCCTCTTTT CCAACGGGTG TGTAGTTCCA 60
TTCTCCACCG CTAGGGCTGC GCTGGGCTCC ATTGGCGAGG TTTTCAAGGC CGCCAGGATG 120
CG 122

(2) INFORMATION ZU SEQ ID NO: 5:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(xi) SEQUENZBESCHREIBUNG: SEQ ID NO: 5:

GGGGGGGAAT TCATGTATAG C 21

(2) INFORMATION ZU SEQ ID NO: 6:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(xi) SEQUENZBESCHREIBUNG: SEQ ID NO: 6:

GGGGGGGATT CAGTGCAGTC A 21

(2) INFORMATION ZU SEQ ID NO: 7:

(i) SEQUENZ CHARAKTERISTIKA:
(A) LÄNGE: 36 Basenpaare
(B) ART: Nukleinsäure
(C) STRANGFORM: Einzel
(D) TOPOLOGIE: linear

(ii) ART DES MOLEKÜLS: DNS (genomisch)

(xi) SEQUENZBESCHREIBUNG: SEQ ID NO: 7:

GGGGGGGAAT CCGGTCAGAA CCTGTGCCTG TGCGAA 36

(2) INFORMATION ZU SEQ ID NO: 8:

(i) SEQUENZ CHARAKTERISTIKA:
(A) LÄNGE: 45 Basenpaare
(B) ART: Nukleinsäure
(C) STRANGFORM: Einzel
(D) TOPOLOGIE: linear

(ii) ART DES MOLEKÜLS: DNS (genomisch)

(xi) SEQUENZBESCHREIBUNG: SEQ ID NO: 8:

GGGGGGGTCG ACCCTAGATC TCTATTACTG CAGGTATTCT TCCGG 45

## Beschreibung

Die vorliegende Erfindung betrifft ein Verfahren zur rekombinanten Herstellung von Proteinen in Hefen.

Die rekombinante Herstellung von Proteinen in Hefen ist bekannt. Heterologe Gene können als intrazelluläre Proteine exprimiert werden oder direkt ins Medium sezerniert werden. Letzteres setzt voraus, daß vor das heterologe Protein bzw. Gen eine Signalsequenz fusioniert wird. In S. cerevisiae wurden die Signalsequenzen der genuinen sekretorischen Proteine für das α-Faktor-Pheromon (Brake A.J., et al., Natl. Acad. Sci. USA 81, 4642-4616 (1984)), die Invertase (Taussig R. & Carlsorn M., Nucleic Acids Res. 11, 1943-1954 (1983)) und die saure Phosphatase (Meyhack B et al., EMBO J. 1, 675-680 (1982)) für die Sekretion heterologer Proteine eingesetzt (Kingsman S., et al., In: Russel G.E. (Ed.) Yeast Biotechnology (pp 113-152). Intercept Ltd., Wimborne, Dorset (1988); Chiron EP 116 201).

Fusionen mit diesem Pre-Pro-Fragment führen auch in anderen Hefen zur Sekretion heterologer Proteine, so daß man von einem ähnlichen Prozessierungs- und Sektretionsmechanismus ausgehen kann (Gellissen G. et. al., Biotech. Adv. 10, 179-189 (1992); Vedvick T. et al., J. Ind. Microbiol. 7, 197-202 (1992)).

In Hefen werden auch die genuinen Signalsequenzen eines heterologen Proteins erkannt.

In der Hefe Hansenula polymorpha wird die Glucoamylase-Leadersequenz (GAM1) aus Schwanniomyces occidentalis als Signalsequenz erkannt und es ist möglich, korrekt prozessierte Glucoamylase zu sezernieren (G. Gellissen et al., Biotechnology 9, 291-295, 1991). Diese Signalsequenz führt aber nicht zur Sekretion Hefefremder Genprodukte, beispielsweise ist die Sekretion des Proteins Hirudin damit nicht möglich.

Aufgabe der vorliegenden Erfindung war es daher, ein Verfahren zur rekombinanten Herstellung von Proteinen, insbesondere von Hefe-fremden, d.h. heterologen Proteinen, in der Hefe Hansenula bereitzustellen, das für eine Vielzahl von Proteinen die effiziente Sekretion und korrekte Prozessierung gewährleistet.

Diese Aufgabe wird gelöst durch ein Verfahren zur rekombinanten Herstellung von Proteinen in Hefe, das dadurch gekennzeichnet ist, daß die Hefe mit einer Expressionskassette transformiert wird, die folgende Strukturelemente codiert enthält:
L - A - P - GEN
wobei
- L:: eine Leadersequenz eines tierischen peptidischen Neurohormons,
- A:: ein eine alpha-Helix-Struktur erzeugender Adaptor,
- P:: ein Prozessierungssignal und
- GEN:: ein Strukturgen für das gewünschte Protein
darstellen.

Als Leadersequenzen sind alle Leadersequenzen von tierischen peptidischen Neurohormonen geeignet. Insbesondere sind solche Leadersequenzen geeignet, die von Neurohormonen aus wirbellosen Tieren, wie Insekten und Mollusken stammen.

Beispiele für solche tierischen peptidischen Neurohormone sind PBAN aus Mais-Ohrwurm (Davis et al., Proc. Natl. Acad. Sci. USA 89, 142-146, 1992), 5-KD-Peptid aus Grashüpfer (Eur. J. Biochem. 187, 249-254, 1990), hyperglykämisches Hormon aus Strandkrabbe (FEBS Letter, 257, 31-34, 1989), Vasotocin aus Kröte (Proc. Natl, Acad. Sci. USA 84, 3043-3046, 1987).

Besonders gut geeignet für das erfindungsgemäße Verfahren ist die Leadersequenz aus dem hyperglykämischen Hormon der Strandkrabbe. Sie besteht aus den Aminosäuren Nr. 1 bis 26 der Sequenz SEQ ID NO:1.

Als Adaptor A sind alle Sequenzen geeignet, die für ein Polypeptid codieren, das eine alpha-Helix-Struktur enthält. Das Vorhandensein einer alpha-Helix-Struktur kann nach dem Algorithmus von Garnier et al. (J. Mol. Biol. 120, 97-120, 1978) ermittelt werden. Besonders leicht läßt sich mit kommerziell erhältlichen Computerprogrammen, die auf diesem Algorithmus basieren, ermitteln, ob eine Polypeptidsequenz eine alpha-Helix-Struktur besitzen sollte.

In der Regel sind alle solche Sequenzen als Adaptor gut geeignet, für die mit dem Computerprogramm "Microgenie"® (Beckmann) im Bereich der Prozessierungsstelle A-P-GEN für eine Peptidsequenz von mindestens vier Aminosäuren für ALPHA ein größerer positiver Wert als für die drei anderen Strukturmöglichkeiten (BETA, TURN, COIL) errechnet wird.

Für die erfindungsgemäße Anwendung kann die Länge der Adaptorsequenz A in großen Bereichen variieren. Sie beträgt in der Regel zwischen fünf und einhundert Aminosäuren.

Bevorzugt wird als Adaptorsequenz A die 38 Aminosäuren lange Sequenz SEQ ID NO:1 Nr. 27-64 verwendet.

Diese Sequenz kann direkt oder besonders bevorzugt nach Verlängerung am C-Terminus um ein bis vier Aminosäuren als Adaptorsequenz verwendet werden. Auch Teile dieser Sequenz, bevorzugt solche die durch N-terminale Verkürzung erhalten werden, sind für das erfindungsgemäße Verfahren gut geeignet.

Man kann beispielsweise auch anhand des oben beschriebenen Computerprogramms die Teile, die besonders zur alpha-Helix-Bildung beitragen, identifizieren und durch Austausch einzelner Aminosäuren noch hinsichtlich der alpha-Helix-Struktur optimieren.

Das Prozessierungssignal P dient der Spaltung des Propeptids in die reife Form. Üblicherweise wird eine Sequenz aus basischen Aminosäuren bevorzugt aus Lys oder Arg, als Prozessierungssignal verwendet. Gut geeignet als Prozessierungssignal ist die KEX2-Erkennungsstelle aus S. cerevisiae, die aus dem Dipeptid Lys - Arg besteht und auch von anderen Hefen erkannt wird. Dieses Dipeptid kann auch in duplizierter Form als Prozessierungssignal verwendet werden. Bevorzugt wird als P die Sequenz Lys - Arg.

Als Strukturgen GEN für das herzustellende Protein können heterologe und homologe Gene verwendet werden. Die Gene können aus den entsprechenden Organismen isoliert oder synthetisch hergestellt werden. Bei chemischer Gensynthese kann gewünschtenfalls auch eine Anpassung der Codon-usage an den Produktionsorganismus erfolgen.

Bevorzugt werden als Strukturgene eukaryotische Gene und virale Gene eingesetzt. Das erfindungsgemäße Verfahren gelingt besonders gut für die Herstellung von Thrombininhibitoren, beispielsweise von Hirudin. Auch für die Herstellung von humanen Polypeptiden beispielsweise von Peptidhormonen, Wachstumsfaktoren, Lymphokinen ist dieses Verfahren gut geeignet.

Die oben genannten Strukturelemente werden in bekannter Weise in der Folge L - A - P - GEN in einer Expressionskassette angeordnet. Die Verknüpfung geschieht üblicherweise durch Ligation kompatibler Restriktionfragmente oder durch chemische Synthese.

Die Expressionskassetten können weiterhin eine Reihe von üblichen Regulationssignalen wie Promotoren, RBS-Bindungsstellen, Terminatoren enthalten, die funktionell mit den erfindungsgemäßen Strukturelementen L - A - P - GEN verbunden werden.

Die Expressionskassette kann Bestandteil eines autonom replizierenden oder auch eines integrativen Vektors sein. Die Konstruktion eines Expressionsvektors unter Verwendung der Expressionskassette ist in Beispiel 1 beschrieben.

Die Hefe wird mit dem entsprechenden Expressionsvektor transformiert. Dies kann beispielsweise nach dem in Beispiel 2 beschriebenen Protokoll geschehen.

Von der so transformierten Hefe werden stabil exprimierende Klone isoliert, die als Produktionsorganismus in dem erfindungsgemäßen Verfahren geeignet sind. Die Produktionsorganismen werden unter üblichen Bedingungen gezüchtet und produzieren je nach gewählten Regulationselementen in konstitutiver oder induzierbarer Weise das gewünschte Protein. Das Protein wird vom Produktionsorganismus in das umgebende Medium sezerniert, von wo es leicht isoliert und gereinigt werden kann.

Die Aufreinigung aus dem Medium geschieht in der Regel, nachdem der Produktionsorganismus abgetrennt worden ist, durch in der Proteinchemie geläufige Reinigungsverfahren.

Das erfindungsgemäße Verfahren liefert korrekt prozessierte reife Proteine ohne die sonst zu beobachtende Fehlprozessierung. Daher führt dieses Verfahren zu einer hohen Ausbeute an reifem Protein und erleichtert die nachfolgenden Reinigungsschritte erheblich. Dieses Verfahren ist daher besonders gut bei der Herstellung von Arzneimitteln auf Basis von Pharmaproteinen einsetzbar.

Die folgenden Beispiele dienen der weiteren Veranschaulichung der Erfindung.

### Beispiel 1

### Konstruktion von Vektoren zur sekretorischen Expression von rekombinanten Proteinen aus dem Hefestamm Hansenula polymorpha

In diesem Beispiel wird die Konstruktion von Expressionsvektoren beschrieben, die bei der erfindungsgemäßen Herstellung rekombinanter Proteine in Hansenula polymorpha zur Anwendung kommen. Die dabei verwendete Expressionskassette besteht u.a. aus folgenden Bestandteilen:
- Leader:: Aminosäure 1-26 von SEQ ID NO: 1
- Adaptor:: Aminosäure 27-64 von SEQ ID NO: 1
- P:: Aminosäure 65-66 von SEQ ID NO: 1
- GEN:: Hirudin-Gen (SEQ ID NO: 2)

Das Konstrukt wurde aus zwei DNA Fragmenten zusammengesetzt. Das erste Fragment setzte sich aus den oben angeführten Leader-Adaptor-Prozessierungssequenzen sowie den 5'-terminalen Nukleotiden des Hirudingens für Aminosäure 1 (Val) bis 7 (Thr) SEQ ID NO: 2 (L-A-P-5'-Hir-Fragment) zusammen. Das zweite Fragment wurde von den restlichen Aminosäuren des Hirudingens (8 (Glu)) bis 65 (Gln), SEQ ID NO: 2) abgeleitet (3'-Hir-Fragment).

Zur Herstellung des L-A-P-5'-Hir-Fragments wurden zwei Oligonukleotide SEQ ID NO: 3 und SEQ ID NO: 4 synthetisiert. Die beiden Oligonukleotide sind an ihren 3'-Enden im Bereich von 20 Nukleotiden überlappend komplementär.

Durch Zugabe von Nukleosidtriphosphaten und Polymerase wurden in einer PCR die DNA-Moleküle in den nicht komplementären einzelsträngigen Abschnitten zum Doppelstrang aufgefüllt. Durch Zugabe von zwei Amplifikationsprimern (SEQ ID NO: 5 und SEQ ID NO:6) und PCR-Amplifikation wurde die DNA vermehrt. Das so entstandene L-A-P-5'-Hir-Fragment wurde am 5'-Ende mit Eco R1 und am 3'-Ende mit Hinf I nachgeschnitten.

Das 3'-Hir-Fragment wurde ausgehend vom bekannten Hirudingen mit Hilfe von zwei synthetischen Amplifikationsprimern (SEQ ID NO: 7 und SEQ ID NO: 8) und PCR-Amplifikation hergestellt.

Das so gewonnene Fragment wurde am 5'-Ende mit Hinf I und dem 3'-Ende mit Sal I nachgeschnitten.

Durch Ligation der 3'-Ende Hinf I-Stelle des L-A-P-5'-Hir-Fragments mit der 5'-Ende Hinf I-Stelle des 3'-Hir-Fragments sowie durch Ligation dieser DNA über Eco RI/Sal I in den Vektor pUC 18 wurde das Konstrukt fertiggestellt.

Aus diesem Konstrukt wurde wiederum das L-A-P-Hir-Fragment als EcoRI/Bgl II-Fragment isoliert und in den entsprechend vorbereiteten H.polymorpha Expressionsvektor pFMD 13025 (Gellissen G. et al., TIBTECH, 10, 413-417, 1992) ligiert. Dabei wird das 5' Ende von L-A-P-Hir an den H.polymorpha-Promotor und das 3' Ende des Fragmentes an den H.polymorpha-Terminator fusioniert. Die Expressionskassette ist nunmehr vollständig und Bestandteil eines Shuttle-Vektors, mit dem zwecks Propagierung sowohl E.coli als auch zwecks Expression des Fremdgens die Hefe H.polymorpha transformiert werden kann.

Die gleiche L-A-P Konstruktion wurde mit dem Gen für den Thrombininhibitor Rhodniin aus Rhodnius prolixus (WO 93/8282) sowie mit dem Gen für den Thrombininhibitor Moubatin aus Ornithodorus moubata (WO 93/9232) fusioniert. Die dabei erhaltenen Expressionskassetten wurden analog zu den Hirudingenfusionen für die Konstruktion von Hansenula polymorpha Expressionsvektoren eingesetzt.

### Beispiel 2

### Transformation von Hansenula polymorpha mit den Expressionsvektoren

Wirtsstamm für die Transformation ist eine durch EMS-Mutagenese erhaltene auxotrophe Mutante: ein Stamm mit einer Defizienz für Orotidin-5'-Phosphat-Dehydrogenase (ura⁻). Die Reversionsrate dieser Uracil-Mutante kann vernachlässigt werden.

Kompetente Zellen dieses Stammes erhielt man folgendermaßen (nach Dohmen et al., Yeast 7, 691-692, 1992):
10 ml Hefe-Vollmedium (YPD) wurden mit dem Wirtsstamm beimpft und über Nacht bei 37°C schüttelnd kultiviert. Diese Vorkultur wurde in 200 ml YPD-Medium überimpft und bei 37°C schüttelnd bis zu einer OD₆₀₀ ₙₘ = 0,6 - 1,0 kultiviert. Die Zellen wurden mit 0,5 ml Volumen Lösung A (1 M Sorbitol, 10 mM Bicin pH 8,35, 3 % Ethylenglycol) bei Raumtemperatur gewaschen und anschließend in 0,02 Volumen der Lösung A resuspendiert.

Nach Zusatz von 11 µl DMSO wurden die Aliquots bei -70°C bis zur Transformation gelagert.

Zwecks Transformation wurden 10 µg Plasmid-DNA und 100 µl kalte 0,1 M Calciumchloridlösung direkt zu den gefrorenen kompetenten Zellen gegeben.

Nach raschem Auftauen bei 37°C wurde jeder Transformationsansatz mit 1,0 ml Lösung B (40 % Polyethylenglycol PEG 3350, 200 mM Bicin pH 8,35) eine Stunde bei 37°C inkubiert. Die Zellen wurden anschließend in 1 ml Lösung C (150 mM NaCl, 10 mM Bicin pH 8,35) gewaschen, in 200 µl Lösung C resuspendiert und auf Selektivmedium (YNB-Glucose, Komplementierung der Uracil-Defizienz durch ura⁺-Expressionsplasmide) plattiert. Die Inkubation erfolgte für 3 - 5 Tage bei 37°C.

### Beispiel 3

### Isolierung mitotisch stabiler Klone

Die rekombinanten Expressionsplasmide, die für die Transformation von H. polymorpha eingesetzt wurden, sind autonom replizierend und können spontan in das Hefegenom integrieren. Dabei bilden sie eine multimere Struktur: die Plasmidmonomere sind "head to tail" miteinander verbunden.

Mehrere Kopien der Expressionskassette tragen daher zur Produktion des rekombinanten Genproduktes bei. Die Produktivität eines rekombinanten Stammes ist in weiten Bereichen linear zur Anzahl integrierter Expressionskassetten. Die multimere Integration der Fremd-DNA in das Hefegenom und die Isolierung mitotisch stabiler Klone wurde folgendermaßen erreicht:

Die Transformanden wurden von den Agarplatten mit Selektivmedium in 3 ml entsprechendem Flüssigmedium angeimpft und passagiert, d.h. über einen Zeitraum von 1-2 Wochen wurde immer wieder in frisches YNB-Glucose-Medium überimpft (50 µl in 3 ml Medium, Kultivierungen bei 37°C). Während dieser Passagierung integrierte die Plasmid-DNA in das Hefegenom, so daß dann mitotisch stabile Klone erhalten wurden.

Die mitotische Stabilität wurde folgendermaßen getestet:
Aus der letzen Passagierungskultur in YNB-Glucose-Medium wurde dreimal für 1-2 Tage in Vollmedium (YPD) überimpft und bei 37°C kultiviert. Anschließend wurde die verdünnte Kultur auf Vollmedium und auf Selektivmedium plattiert. Mitotisch stabile Transformanden ergeben auf beiden Medien ungefähr gleiche Kolonienzahl. Es können so mitotisch stabile Subtransformanden isoliert werden (Z.A. Janowicz et al., Yeast 7, 431-443 (1991).

### Beispiel 4

### Expression von Fremdgen

Für Expressionsstudien wurden die passagierten Transformanden in 3ml YNB-Medium mit 1 % Glycerin oder 1 % Methanol angeimpft, um MOX-bzw. FMD-Promotoren zu induzieren. Die Zellen wurden nach zweitägiger Kultivierung bei 37°C abzentrifugiert und der Kulturüberstand auf Fremdprotein getestet (Western-Blot, ELISA, Aktivitätstest).

Mit effizient sezernierenden mitotisch stabilen Transformanden wurden 50 ml synthetisches Medium mit 1,5 % Glycerin im 500 ml Schikanen-Erlenmeyer-Kolben angeimpft und bis zu einer OD₆₀₀ ₙₘ = 10 inkubiert. HPLC-Analysen von entsprechenden Kulturüberständen zeigten, daß die Hirudinvariante bei Verwendung der Sequenz SEQ ID NO: 1 Nr. 1 bis 64 als Leader-Adaptor vollständig korrekt prozessiert wird.

### Beispiel 5

### Fermentation von rekombinanten Hefe-Stämmen

Die rekombinanten Hefestämme wurden in synthetischen Medien (doppelt konzentriertes YNB-Medium 2,8 g/L (Difco) mit 10 g/L Ammoniumsulfat) fermentiert, die zu Beginn der Fermentation entweder komplett vorgelegt worden waren oder im Laufe der Fermentation nachgefüttert wurden.

Als Kohlenstoffquellen wurden Glycerin und Methanol oder Gemische aus Glycerin und Methanol eingesetzt. Die Fermentation wurde mit Glycerin als einziger Kohlenstoffquelle gestartet (≥ 1 % Glycerin Endkonzentration im Fermenter während der Anwachsphase).

Nach der Sterilisation des Mediums wurde mit 1 L Vorkultur so angeimpft, daß sich eine Start OD₆₀₀ ₙₘ von ca. 1 ergab.

Der Fermentationsverlauf war zweiphasig: auf eine Anwachsphase mit höherer Glycerinkonzentration (1 %) folgte eine Produktionsphase mit geringerer Glycerinkonzentration (<0,5 %) oder konstanter Methanolkonzentration (1 %) oder einer Mischung aus Glycerin und Methanol (0,1 - 0,4 % Glycerin und 0,2 - 1,0 % Methanol).

Die Kohlenstoffquelle wurde gegebenenfalls über verschiedene Steuerungsmöglichkeiten (kontinuierlich oder pO2 gekoppelt) nachgefüttert.

Im Verlauf der Fermentation wurde Ammoniumsulfat bis zu einer Endkonzentration von 5 g/L , Thiamin bis zu einer Endkonzentration von 0,1 g/L und Biotin bis zu einer Endkonzentration von 0,3 mg/L zugegeben.

Der pH-Wert der Fermentation wurde durch Zugabe von Ammoniakwasser bei 4,0 konstant gehalten; die Fermentationstemperatur betrug 37°C.

Die so fermentierten rekombinanten Hefe-Stämme lieferten ein zu 100 % korrekt prozessiertes Genprodukt (Hirudin).

## Patentansprüche

1. Verfahren zur rekombinanten Herstellung von Proteinen in Hefe, dadurch gekennzeichnet, daß die Hefe mit einer Expressionskassette transformiert wird, die folgende Strukturelemente codiert enthält:
L - A - P - GEN
wobei
L: eine Leadersequenz eines tierischen peptidischen Neurohormons,
A: ein eine alpha-Helix-Struktur erzeugender Adaptor,
P: ein Prozessierungssignal und
GEN: ein Strukturgen für das gewünschte Protein
darstellen.

2. Verfahren nach Anspruch 1, dadurch gekennzeichnet, daß
L: für die Aminosäuresequenz 1-26 von SEQ ID NO:1,
A: für die Aminosäuresequenz 27-64 von SEQ ID NO:1 und
P: für die Aminosäuresequenz 65-66 von SEQ ID NO:1
steht.

3. Verfahren nach Anspruch 1 oder 2, dadurch gekennzeichnet, daß als Hefe eine Hefe der Gattung Hansenula, Saccharomyces, Kluyveromyces oder Pichia verwendet wird.

## Claims

1. A process for the recombinant production of proteins in yeast, which comprises transforming the yeast with an expression cassette which comprises the following structural elements encoded:
L - A - P - GEN
where
L is a leader sequence of an animal peptide neurohormone,
A is an adaptor producing an alpha-helix structure,
P is a processing signal and
GEN is a structural gene for the required protein.

2. A process as claimed in claim 1, wherein
L is amino acid sequence 1-26 of SEQ ID NO: 1,
A is amino acid sequence 27-64 of SEQ ID NO: 1 and
P is amino acid sequence 65-66 of SEQ ID NO: 1.

3. A process as claimed in claim 1 or 2, wherein a yeast of the genus Hansenula, Saccharomyces, Kluyveromyces or Pichia is used as yeast.

## Revendications

1. Procédé pour la préparation recombinante de protéines dans de la levure, caractérisé par le fait que la levure est transformée avec une cassette d'expression qui contient sous forme codée les éléments structuraux suivants:
L - A - P - GEN
où
L: désigne une séquence de tête d'une neurohormone peptidique animale,
A: représente un adaptateur produisant une structure en hélice alpha,
P: désigne un signal de traitement et
GEN: représente un gène de structure pour la protéine souhaitée.

2. Procédé selon la revendication 1, caractérisé par le fait que
L: désigne la séquence d'acides aminés 1-26 de SEQ ID NO:1,
A: désigne la séquence d'acides aminés 27-64 de SEQ ID NO:1 et
P: désigne la séquence d'acides aminés 65-66 de SEQ ID NO:1.

3. Procédé selon la revendication 1 ou 2, caractérisé par le fait qu'on utilise comme levure une levure de l'espèce Hansenula, Saccharomyces, Kluyveromyces ou Pichia.
